# EUROPÄISCHE PATENTSCHRIFT

(11) **EP 1 946 706 B1**
(45) Veröffentlichungstag und Bekanntmachung des Hinweises auf die Patenterteilung: **01.12.2010**
(21) Anmeldenummer: 07000834.7
(22) Anmeldetag: 17.01.2007
(51) Int. Cl.: A61B 17/32, H02P 6/06, A61B 19/00

(54) **Medizinischer Handgriff**
Medical handle
Poignée médicale

(43) Veröffentlichungstag der Anmeldung: 23.07.2008
(73) Patentinhaber: W & H Dentalwerk Bürmoos GmbH, 5111 Bürmoos (AT)
(72) Erfinder: Schneider, Rainer, 5120 St. Pantaleon (AT); Schröck, Rainer, 5112 Lamprechtshausen (AT); Pletscher, Walter, 8762 Oberzeiring (AT); Ott, Josef, 8740 Zeltweg (AT)

(56) Entgegenhaltungen:
- WO-A-2007/002180
- US-A- 5 270 622
- US-A- 6 013 991
- US-A1- 2001 043 806
- US-A1- 2005 107 814

## Beschreibung

Die vorliegende Erfindung betrifft einen medizinischen Handgriff mit einer Antriebseinheit zum Antrieb eines medizinischen Werkzeugs nach dem Oberbegriff des Anspruchs 1, wie in WO-2007/002180 offenbart.

Derartige Handgriffe sind heute weit verbreitet und werden für eine große Anzahl unterschiedlicher medizinischer, chirurgischer oder kosmetischer Eingriffe verwendet. Die Antriebseinheit dieser Handgriffe umfasst oftmals einen bürstenlosen Elektromotor, der eine Rotationsbewegung erzeugt, die auf ein Behandlungswerkzeug übertragen wird. Gegebenenfalls kann die Antriebseinheit des Weiteren eine oder mehrere Wellen zur Übertragung der Rotationsbewegung, Getriebe oder Vorrichtungen zur Umwandlung der Rotationsbewegung in eine andere Bewegungsart umfassen.

Die bürstenlosen Elektromotore der Handgriffe sind üblicherweise steuerbar, meist kann der Anwender über ein Stellorgan und eine Steuervorrichtung zumindest die Drehzahl des Motors verändern. Der grundsätzliche Aufbau einer derartigen Steuervorrichtung ist schematisch in der US 5,270,622, Fig. 1 dargestellt. Dem bürstenlosen Elektromotor mit einem Stator und mit einer Rotoreinheit mit einem Magnetelement ist eine Sensorik zur Erkennung der Ausrichtung des Magnetelements zugeordnet. Die Signale der Sensorik werden der Steuervorrichtung zugeleitet, die in Abhängigkeit der Drehzahlvorgabe des Anwenders und der Sensorsignale Antriebssignale an den Motor abgibt. Die Sensorik ist üblicherweise als Teil des Stators ausgeführt und mit diesem fest verbunden, meist vergossen. Die Sensorik zur Erkennung der Ausrichtung des Magnetelements und der bürstenlosen Elektromotor sind dabei räumlich getrennt von der Steuervorrichtung angeordnet und durch Leitungen zur Signalweiterleitung miteinander verbunden.

Der Nachteil einer derartigen Ausführung liegt in ihrem komplizierten Aufbau, der während der Montage mehrere Schritte umfasst, insbesondere die Befestigung der Sensorik am Stator, den Einbau der Steuervorrichtung in den Handgriff und die Verbindung der Sensorik mit der Steuervorrichtung über mehrere Leitungen.

Da derartige medizinische Handgriffe aus hygienischen Gründen nach ihrer Verwendung sterilisiert werden müssen, ist es notwendig, die elektrischen und elektronischen Bauteile vor den aggressiven Umgebungsbedingungen, die während der Sterilisation herrschen, zu schützen. Dies erfolgt üblicherweise durch Vergießen der Bauteile mit Harzen und / oder deren Einkapselung in hermetisch abgeschlossenen Gehäusen. Die Eintrittsstellen von Leitungen und Kabeln in das Vergussmaterial oder in das Gehäuse stellen dabei Schwachstellen dar, an denen Undichtheiten entstehen und in weiterer Folge Wasserdampf oder korrosiv wirkende Medien wie Reinigungsmittel bis zu den elektrischen und elektronischen Bauteilen vordringen und diese beschädigen können, so dass es in nachteiliger Weise zu einem verfrühten Ausfall des Handgriffs kommt und aufwendige Reparaturmaßnahmen oder der vollständige Austausch des Geräts notwendig werden.

Schließlich besteht beim Verbinden der Sensorik mit dem Elektromotor durch Vergießen mit Harz immer wieder das Problem, dass die Isolierung der Signalleitung zwischen der Sensorik und der Steuervorrichtung schmilzt, wodurch die einwandfreie Funktion des Handgriffs beeinträchtigt wird. Damit wird zusätzlicher Prüfaufwand nach der Montage notwendig, der die Unversehrtheit der Leitungen sicherstellt, bzw. wenn die Leitungen beschädigt wurden, ist eine Reparatur oder ein Austausch der Leitungen erforderlich.

Der vorliegenden Erfindung liegt daher die Aufgabe zugrunde einen medizinischen Handgriff zu schaffen, der die oben angeführten Nachteile nicht aufweist. Der Handgriff soll insbesondere einfacher zu montieren und resistenter gegenüber den bei der Sterilisation herrschenden Umgebungsbedingungen sein als bekannte Handgriffe.

Diese Aufgabe wird gemäß der vorliegenden Erfindung durch einen medizinischen Handgriff mit den Merkmalen des Anspruchs 1 gelöst. Besonders vorteilhafte Ausführungsformen sind in den Unteransprüchen angeführt.

Bei dem erfindungsgemäßen medizinische Handgriff sind die Sensorik zur Erkennung der Ausrichtung, i.e. des Drehwinkels, des zumindest einen Magnetelements (Motorsensorik) und die Steuer- und / oder Regelschaltung in einem gemeinsamen Steuermodul zusammengefasst. Die Zusammenführung der Motorsensorik und der Steuer- und / oder Regelschaltung zu einem einstückigen Bauteil vereinfacht die Montage und insbesondere den Einbau in den Handgriff merklich. Um die Zusammenfassung zu einem einstückigen Steuermodul und dessen Einsatz in dem medizinischen Handgriff zu ermöglich, ist es zusätzlich notwendig, sowohl den Aufbau des Handgriffs zu modifizieren als auch die Sensorik, die Steuer- und / oder Regelschaltung und gegebenenfalls weiterer in dem Steuermodul eingebaute Bauelemente entsprechend anzuordnen. Weitere in dem Steuermodul eingebaute Bauelemente können insbesondere durch zumindest Teile der Schaltung zur Auswahl der Antriebsdrehzahl und / oder zumindest Teile der Schaltung zur Auswahl der Drehrichtung des bürstenlosen Elektromotors und / oder elektrische Kontakte zu einer Energiequelle und / oder Kontakte zur Verbindung des Steuermoduls, insbesondere der Steuer- und / oder Regelschaltung mit der Antriebseinheit, und / oder zumindest Teile einer Schaltung zur Temperaturüberwachung Wärme abgebender Bauteile des Handgriffs, zum Beispiel von Akkumulatoren, und / oder zumindest Teile einer Schaltung zur Spannungsüberwachung spannungsführender Bauteil des Handgriffs, zum Beispiel zur Unterspannungsabschaltung von Akkumulatoren, gebildet sein.

Der Aufbau des Handgriffs muss dahingehend verändert werden, dass das zumindest eine Magnetelement, dessen Position oder Ausrichtung durch die Sensorik bestimmt wird, derart im medizinischen Handgriff angeordnet ist, dass zumindest ein Teil des Steuermoduls in unmittelbarer Nähe des zumindest einen Magnetelements positionierbar ist. Das Magnetelement kann dabei entweder durch den Rotormagneten selbst oder durch einen separaten Sensormagneten, der in bekannter Weise mit dem Rotormagneten rotiert, gebildet werden.

In einem ersten Ausführungsbeispiel ist der medizinische Handgriff mit einem Griffabschnitt und einem dazu gewinkelt angeordneten Funktionsabschnitt versehen, der zum Beispiel die Werkzeugaufnahme, eine Beleuchtungsvorrichtung, Medienleitungen und andere Funktionselemente beinhaltet, wobei das zumindest eine Magnetelement in einem der beiden Abschnitte angeordnet ist und das Steuermodul zumindest teilweise auch in jenem Abschnitt aufgenommen ist, in dem das zumindest eine Magnetelement nicht angeordnet ist. Bevorzugt ist auch der bürstenlose Motor im Funktionsabschnitt aufgenommen, wobei das zumindest eine Magnetelement in einem Bereich des Funktionsabschnitts angeordnet ist, an den der Griffabschnitt anschließt, so dass das Steuermodul zumindest teilweise im Griffabschnitt aufgenommen ist. Ein Vorteil dieses Ausführungsbeispiels besteht darin, dass zumindest Teile von Schaltungen, die mit Stellorganen zusammenwirken, die am Griffabschnitt angeordnet oder befestigt sind, in das Steuermodul integriert werden können, so dass bevorzugt alle Schaltungen in einem einzigen, kompakten Steuermodul vereint sind. Bei diesen zusätzlichen Schaltungen kann es sich zum Beispiel um eine Schaltung zur Auswahl der Antriebsdrehzahl oder eine Schaltung zur Auswahl der Drehrichtung des bürstenlosen Elektromotors handeln. Ein weiterer Vorteil dieser Ausführungsform liegt in der hervorragenden Ausnutzung des vorhandenen Raums im Inneren des Handgriffs, insbesondere wenn das Steuermodul eine längliche, schmale, bevorzugt im Wesentlichen quaderförmige Form aufweist, so dass es problemlos im Griffabschritt des Handgriffs verbaut werden kann.

In einem zweiten Ausführungsbeispiel weist der medizinische Handgriff eine Werkzeugaufnahme für das medizinische Werkzeug oder eine Anschlussvonichtung für eine Werkzeugaufnahme auf und die Rotoreinheit ist mit einem Rotor und einem Sensormagneten versehen, wobei der Sensormagnet und das Steuermodul zwischen dem Rotor und der Werkzeugaufnahme bzw. der Anschlussvorrichtung angeordnet sind. Bevorzugt handelt es sich bei diesem Handgriff um ein längliches, im Wesentlichen rohrförmiges Instrument mit einem ersten Ende mit der Werkzeugaufnahme und einem zweiten, gegenüberliegenden Ende Ein derartiger Handgriff hat im Gegensatz zum Handgriff des ersten Ausführungsbeispiels keinen separaten Griffabschnitt, in dem das Steuermodul zumindest teilweise aufnehmbar ist. Bei diesen länglichen Handgriffen ist aus montagetechnischen Gründen der Sensormagnet üblicherweise am zweiten Ende angeordnet, d.h. zwischen dem Rotor und dem instrumentenende. Werden, wie erfindungsgemäß vorgeschlagen, die Sensorik zur Erkennung der Ausrichtung des zumindest einen Magnetelements und die Steuer- und / oder Regelschaltung in einem gemeinsamen Steuermodul zusammengefasst, so bleibt in diesem Endbereich des Handgriffs, wo sich der Sensormagnet befindet, zu wenig Platz um dort zusätzlich das Steuermodul unterzubringen. Erst durch die Anordnung des Sensormagneten zwischen dem Rotor und der Werkzeugaufnahme bzw. der Anschlussvorrichtung wird ausreichend Platz für das Steuermodul geschaffen, um das Steuermodul in unmittelbarer Nähe zum Sensormagneten positionieren zu können.

Die Sensorik zur Erkennung der Ausrichtung des zumindest einen Magnetelements muss derart in dem Steuermodul angeordnet sein, dass sie in unmittelbarer Nähe des zumindest einen Magnetelements positionierbar ist. Ist das Steuermodul länglich, zum Beispiel im Wesentlichen quaderförmig, gerade oder gebogen ausgebildet, so dass es ein erstes und zweites Ende aufweist, so ist es von Vorteil, die Sensorik unmittelbar an einem der beiden Enden des Steuermoduls anzubringen, wobei das Ende mit der Sensorik, wenn das Steuermodul in den medizinischen Handgriff eingebaut ist, in unmittelbarer Nähe zum zumindest einen Magnetelement angeordnet ist. Falls die Platzverhältnisse im Inneren des Handgriffs dies erfordern, kann das Steuermodul jedoch auch gewinkelt ausgeführt sein, zum Beispiel T-formig oder L-förmig, und zwei oder mehrere miteinander verbundene Arme und auch mehr als zwei Enden aufweisen. Auch bei diesen Ausführungsformen ist es von Vorteil, die Sensorik unmittelbar an einem dieser Enden anzubringen und das Ende mit der Sensorik wiederum in unmittelbarer Nahe zum zumindest einen Magnetelement anzuordnen. Alternativ kann das Steuermodul in Wesentlichen U-förmig geformt sein, wobei die Sensorik zum Beispiel an der Außenseite des Querbalkens, der die beiden gleich oder unterschiedlich langen Schenkel des U-förmigen Steuermoduls verbindet, vorgesehen ist, so dass sich die Sensorik ungefähr in der Mitte des Steuermoduls befindet. Der Querbalken wird in unmittelbarer Nähe zum zumindest einen Magnetelement angeordnet. Auf diese Weise wird es insbesondere in länglichen Handgriffen möglich, die Sensorik in unmittelbarer Nähe des zumindest einen Magnetelements zu positionieren, während alle anderen Schaltungen über den länglichen Innenraum des Handgriffs verteilt sind. Selbstverständlich sind die angeführten Ausformungen des Steuermoduls und der Anordnung der Sensorik nur Beispiele, die ein Fachmann in Anpassung an die vorhandenen raumlichen Verhältnisse im Inneren des Handgriffs entsprechend modifizieren kann.

Um die Bestimmung der Ausrichtung des zumindest einen Magnetelements durch die Sensorik noch zusätzlich zu verbessern, wird in einem weiteren Ausführurigsbeispiel das Steuermodul mit einer definierten Außenform versehen, so dass die Anordnung des Steuermoduls in unmittelbarer Nähe zum zumindest einen Magnetelement noch zusätzlich erleichtert und begünstigt wird. Bevorzugt hat das Steuermodul einen kreisbogenförmigen oder stufenförmigen Rücksprung oder eine Bohrung zur zumindest teilweisen Aufnahme des zumindest einen Magnetelements. Besonders bevorzugt ist die Sensorik unmittelbar anschließend an den Rücksprung oder die Bohrung angebracht.

Zur besseren Handhabung und zwecks einfacherem Einbau in den Handgriff weist das Steuermodul bevorzugt eine Trägervorrichtung auf, insbesondere eine Trägerplatte oder eine Platine, auf der zumindest die Steuer- und / oder Regelschaltung und die Sensorik, besonders bevorzugt auch zumindest Teile weiterer Schaltungen befestigt sind.

Alternativ oder zusätzlich kann das Steuermodul zumindest teilweise in einem Gehäuse aufgenommen sein, wobei bevorzugt das Gehäuse so beschaffen ist, dass die sich im Inneren des Gehäuses befindlichen Bauelemente durch die während eines Reinigungs- oder Sterilisationsvorgangs herrschenden Umgebungsbedingungen in ihrer Funktion nicht beeinträchtigt werden. Zu diesen Bauelementen können neben der Steuer- und / oder Regelschaltung und der Sensorik die schon erwähnten Teile der Schaltung zur Auswahl der Antriebsdrehzahl oder zur Auswahl der Drehrichtung, elektrische Kontakte zu einer Energiequelle, Kontakte zur Verbindung des Steuermoduls, insbesondere der Steuer- und / oder Regelschaltung mit der Antriebseinheit, die Trägervorrichtung oder Kühlelemente zur Ableitung der während des Betriebs des Steuermoduls entstehenden Wärme gehören.

Die Anforderungen denen das Gehäuse während eines Reinigungs- oder Sterilisationsvorgangs widerstehen muss, sind bekannt, so dass darauf nicht im Detail eingegangen wird. Insbesondere muss das Gehäuse je nach Art der Sterilisation oder Reinigung so beschaffen sein, dass es für einen Zeitraum von in etwa zumindest zehn Minuten Temperaturen von zumindest 120°C, Druckschwankungen von in etwa 3 bar oder einer chemisch aggressiven und korrosiven Umgebung standhält. Das Gehäuse kann zum Beispiel aus geeigneten hitzebeständigen Kunststoffen, Harzen oder Metallen hergestellt sein und muss einen hermetisch abgeschlossenen Innenraum aufweisen, so dass Wasserdampf oder Reinigungsmittel auch nach oftmaligem Reinigen oder Sterilisieren davon abgehalten werden, in den Innenraum einzudringen. Als Gehäuse wird auch das Einbetten des Steuermoduls in ein Vergussmaterial, zum Beispiel in ein Kunstharz, insbesondere Epoxyd- oder Silikonharze verstanden.

Ein weiterer montagetechnischer Vorteil des Zusammenfassens der Sensorik und der Steuer- und / oder Regelschaltung in einem gemeinsamen Steuermodul besteht in der einfachen Ausrichtung der Sensorik auf das zumindest eine Magnetelement, das zum Beispiel durch einen Sensormagneten gebildet ist. Die exakte Ausrichtung der Sensorik ist von großer Bedeutung für das Betriebsverhalten der Antriebseinheit, insbesondere für die genaue Ansteuerung und Bestromung des bürstenlosen Elektromotors. Die exakte Anordnung der Sensorik in Bezug auf das zumindest eine Magnetelement wird durch eine genaue, definierte Beabstandung der Sensorik mit zumindest einem weiteren, in dem Steuermodul vorgesehenen Bauteil erreicht. Bevorzugt werden dafür Kontakte, die das Steuermodul mit anderen Komponenten im Handgriff verbinden bzw. über die das Steuermodul mit anderen Komponenten verbindbar ist, verwendet, besonders bevorzugt jene Kontakte, die das Steuermodul mit der Antriebseinheit verbinden. Diese Kontakte können zum Beispiel als Steckkontakte ausgebildet sein, so dass beim Einbau des Steuermoduls in den Handgriff, insbesondere durch das Zusammenstecken der Kontakte nicht nur die Verbindung des Steuermoduls mit der Antriebseinheit bewirkt wird, sondern gleichzeitig auch die Anordnung und exakte Ausrichtung der Sensorik in Bezug auf das zumindest eine Magnetelement.

Die Erfindung wird nachfolgend anhand bevorzugter Ausführungsbeispiele und Bezug nehmend auf die beigefügten Zeichnungen erläutert:
Figur 1 zeigt einen erfindungsgemäßen Handgriff.
Figur 2 zeigt eine Schnittdarstellung entlang der Linie B - B in der Figur 1 durch den Funktionsabschnitt, das Magnetelement und einen Teil des Steuermoduls.
Figur 3 zeigt eine alternative Ausführungsform des Steuermoduls.
Figur 4 zeigt einen Längsschnitt durch eine Ausführungsform des Steuermoduls.

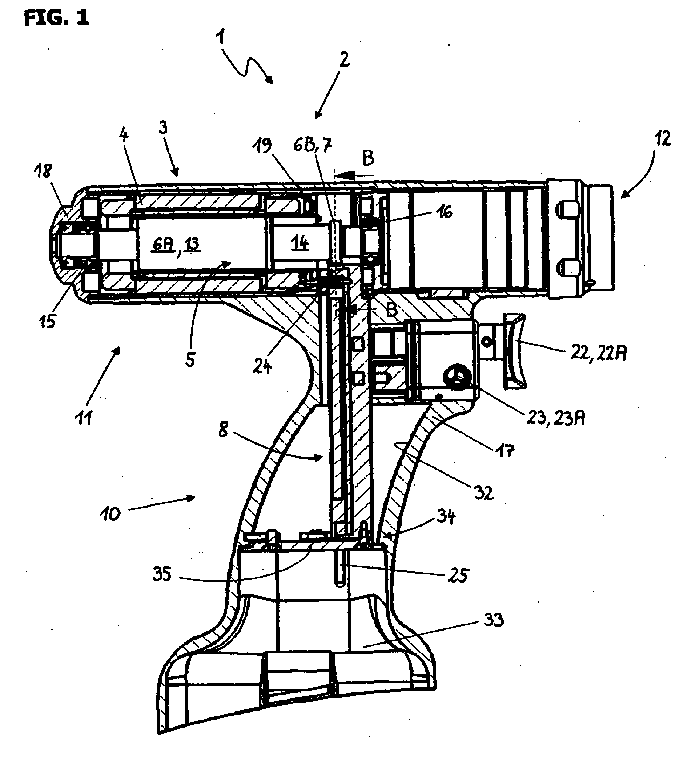

Der in Figur 1 dargestellte Handgriff 1 ist als pistolenförmiges, eine ein- oder mehrteilige Außenhülse 17 umfassendes Instrument ausgebildet, das aus einem Funktionsabschnitt 11 und einem gewinkelt dazu angeordneten Griffabschnitt 10 besteht. Die Erfindung ist jedoch nicht auf diese Handgriffform beschränkt, sondern ist auf andere Handgriffformen in gleicher Weise anwendbar. Im Funktionsabschnitt 11 ist die Antriebseinheit 2, die zumindest einen bürstenlosen Elektromotor 3 mit einem Stator 4 und einer Rotoreinheit 5 umfasst, und die Werkzeugaufnahme oder eine Anschlussvorrichtung 12 zur Verbindung des Handgriffs 1 mit einer Werkzeugaufnahme enthalten. An jenem Ende des röhren- oder hülsenförmig ausgebildeten Funktionsabschnitts 11, das dem Ende mit der Werkzeugaufnahme oder der Anschlussvorrichtung 12 gegenüber liegt, ist eine Abschlusskappe 18 vorgesehen, die den Funktionsabschnitt 11 verschließt und an Schultern und Rücksprüngen im Inneren des Handgriffs angeordnete Bauteile aufnimmt oder lagert.

In bekannter Weise können sowohl die Antriebseinheit 2 als auch der Funktionsabschnitt 11 weitere Bauteile enthalten, so zum Beispiel Getriebe, Vorrichtungen zur Wandlung der rotierenden Bewegung des Elektromotors 3 in eine andere Bewegungsart, zum Beispiel in eine oszillierende, kreisbogenförmige oder Hubbewegung, Beleuchtungsvorrichtungen mit Lichtquellen und / oder Lichtleitern, Medienleitungen, zum Beispiel für Flüssigkeiten oder Gase, Speicher zur Speicherung von Daten, zum Beispiel Betriebsdaten, Sensoren, zum Beispiel für die Werkzeugerkennung etc. Die Werkzeugaufnahme oder die Anschlussvorrichtung 12 zur Verbindung des Handgriffs 1 mit einer Werkzeugaufnahme sind bevorzugt lösbar ausgeführt, so dass unterschiedlichste Werkzeuge, wie zum Beispiel rotierende Bohrer, Sägeblatter, Shaver, Reamer, Feilen etc. an den Handgriff 1 anschließbar sind.

Da bürstenlosen Elektromotore bekannt sind, wird im Weiteren auf den Aufbau des Motors 3 nur so weit eingegangen, wie dies für das Verständnis der Erfindung notwendig ist. Der Elektromotor 3 ist in einer Motorhülse 19 aufgenommen. Die Rotoreinheit 5 besteht aus dem Rotor 13, zumindest einem Magnetelement und einer Rotorwelle 14. Der Rotor 13 kann dabei um die Rotorwelle 14 angebracht und an dieser befestigt sein oder er kann selbst die Rotorwelle 14 bilden oder als Teil der Rotorwelle 14 ausgebildet sein. Die Rotorwelle 14 ist in einem oder mehreren Lagern, bevorzugt Kugellagern 15, 16 gelagert. Der Rotor 13 ist magnetisch oder mit Magneten versehen, so dass er ein erstes Magnetelement 6A bildet. Ein zweites Magnetelement 6B ist als Sensormagnet 7 ausgebildet und auf der Rotorwelle 14, vom Rotor 13 beabstandet befestigt. Der Sensormagnet 7 rotiert mit dem Rotor 13 und wird in bekannter Weise zu Bestimmung der Ausrichtung, d.h. des Drehwinkels des ersten Magnetelements 6A verwendet, so dass eine exakte Ansteuerung und Bestromung des Elektromotors 3 erfolgt.

Der Griffabschnitt 10 weist eine ergonomische Außenform auf, so dass auch längeres Halten des Handgriffs 1 beim Anwender keine zu schnelle Ermüdung der Hand und des Arms verursacht. Am Griffabschnitt 10 ist ein erstes Stellorgan 22 mit einem ersten verschiebbaren Taster 22A angeordnet, über das der Anwender die Drehzahl des Elektromotors 3 steuern kann. Über ein zweites Stellorgan 23 mit einem quer zum Taster 22 verschiebbaren Schaltstift 23A kann der Anwender die Drehrichtung des Elektromotors 3 einstellen. Taster 22A und Schaltstift 23A sind über Öffnungen in der Außenhülse 17 teilweise in Handgriff 1 aufgenommen. Taster 22A und Schaltstift 23A sind jeweils Schaltungen, die im Handgriff 1 angeordnet sind, zur Generierung eines Schalt- oder Steuersignals zugeordnet.

Der Griffabschnitt 10 ist über einen Großteil seiner Länge hohl ausgebildet, so dass in diesem Hohlraum 32 verschiedene Bauteile und / oder Zubehör des Handgriffs 1 fix aufgenommen und lösbar eingesetzt sind, insbesondere eine oder mehrere Batterien oder Akkumulatoren 33 zur Energieversorgung des Handgriffs 1. Die Batterien oder Akkumulatoren 33 sind über elektrische Leitungen oder Kontakte mit allen Verbrauchern im Handgriff 1 verbunden. Zum Verschluss des Hohlraums 32 und zur Fixierung der Batterien oder Akkumulatoren 33 weist der Griffabschnitt 10 an seinem freien Ende eine Verschlussvorrichtung auf, zum Beispiel einen Verschlussdeckel. Alternativ kann die Energieversorgung des Handgriffs 1 auch über eine externe, außerhalb des Handgriffs 1 vorgesehene Energiequelle erfolgen, insbesondere über den Anschluss an ein Stromversorgungsnetz.

Zumindest teilweise ist im Hohlraum 32 des Griffabschnitts 10 auch ein Steuermodul 8 aufgenommen, in dem zumindest die Sensorik 9 zur Bestimmung der Ausrichtung, i.e. des Drehwinkels des Sensormagneten 7 und des Rotors 13 und die Steuer- und / oder Regelschaltung zur Steuerung und / oder Regelung der Antriebseinheit 2 zusammengefasst sind. Die Steuer- und / oder Regelschaltung erzeugt in bekannter Weise ein Antriebssignal zum Antrieb des Elektromotors 3, wobei sie dafür von der Sensorik 9 Messsignale über einen oder mehrere Antriebsparameter des Elektromotors 3 erhält, insbesondere über dessen Drehzahl, diese Messsignale mit einem Vorgabewert vergleicht und abhängig von der Abweichung zwischen dem Messsignal und dem Vorgabewert das Antriebssignal variiert. Die Sensorik 9 kann alle bekannten Sensoren zur Detektion der Stärke eines Magnetfelds umfassen, insbesondere Hallsensoren 37 oder Reed-Kontakte.

Das Steuermodul 8 ist mittels einer oder mehrerer Befestigungsvorrichtungen im Handgriff befestigt. Eine erste Befestigungsvorrichtung 34 umfasst eine Trennplatte 35, die zwischen den Akkumulatoren 33 und dem Steuermodul 8 vorgesehen ist und über eine oder mehrere Schrauben mit dem Handgriff 1 und dem Steuermodul 8 verbunden ist. In der Trennplatte 35 sind mehrere Öffnungen enthalten, durch die Versorgungsleitungen oder Kontaktelemente 25 verlaufen, die die Verbraucher im Handgriff 1 mit den Akkumulatoren 33 verbinden. Als eine zweite Befestigungsvorrichtung für das Steuermodul 8 können die Kontaktelemente 24, die das Steuermodul 8 mit der Antriebseinheit 2 verbinden und über die insbesondere die Antriebssignale an den Elektromotor 3 geleitet werden, dienen, wobei die Kontakte 24 bevorzugt als Steckkontakte ausgebildet sind.

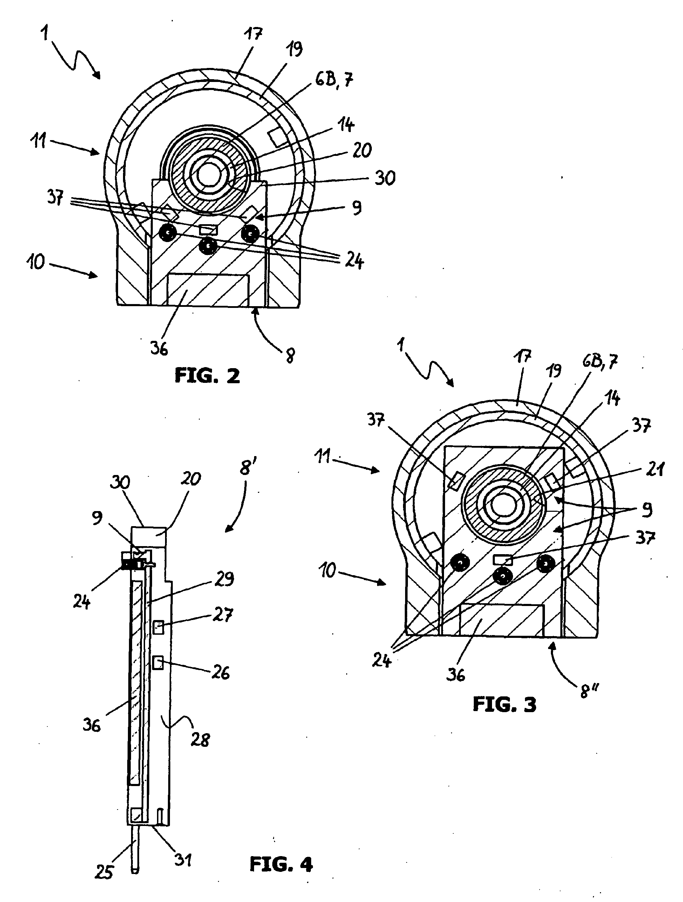

In Figur 4 ist ein bevorzugtes Ausführungsbeispiel eines Steuermoduls dargestellt. Dieses Steuermodul 8' ist länglich, im Wesentlichen quaderförmig ausgebildet, mit einem ersten Ende 30 und einem zweiten Ende 31 und beinhaltet neben der Sensorik 9 zur Bestimmung der Ausrichtung des zumindest einen Magnetelements 6A, 6B und des Rotors 13 und der Steuer- und / oder Regelschaltung zur Steuerung und / oder Regelung der Antriebseinheit 2 mehrere weitere Bauteile: Mit 26 ist ein Teil der Schaltung bezeichnet, über die die Drehrichtung des Elektromotors 3 verändert werden kann. Die Schaltung 26 umfasst bevorzugt einen Sensor zur Bestimmung der Stärke eines Magnetfelds, sie kann jedoch selbstverständlich auch aus anderen Sensoren aufgebaut sein, wenn andere Signalgeber verwendet werden. Zum Umschalten vom Rechts- in den Linkslauf oder umgekehrt verschiebt der Anwender Schaltstift 23A und den damit verbundenen Magneten von einer ersten in eine zweite Position, in welcher der Schaltstift 23A verharrt. Der Sensor zur Bestimmung der Stärke des Magnetfelds, insbesondere ein Reed-Kontakt oder ein Hall-Sensor sind versetzt zum Schaltstift 23A und dem Magneten angeordnet, so dass sich durch deren Verschiebung die Magnetfeldstärke verändert. Der Hall-Sensor sendet ein Messsignal in Abhängigkeit der gemessenen Magnetfeldstärke an die Schaltung 26, die im Folgenden die entsprechende Drehrichtung des Elektromotors 3 auswählt.

Bezugszeichen 27 ist einem Teil jener Schaltung zugeordnet, die dem Anwender die Drehzahlvorgabe für den Elektromotor 3 ermöglicht. Die Schaltung 27 umfasst bevorzugt einen Sensor zur Bestimmung der Stärke des Magnetfelds, insbesondere ein Reed-Kontakt oder ein Hall-Sensor, sie kann jedoch selbstverständlich auch aus anderen Sensoren aufgebaut sein, wenn andere Signalgeber verwendet werden. Stellorgan 22 ist mit einem Magneten verbunden, dessen Abstand durch das Verschieben des Tasters 22A zum Sensor veränderbar ist. Der Sensor und Schaltung 27 bestimmen wiederum die Magnetfeldstärke und generieren ein entsprechendes Signal, das an die Steuer- und / oder Regelschaltung zur Steuerung und / oder Regelung der Antriebseinheit 2 geleitet wird und als Vorgabewert für die Drehzahl dient.

Die Schaltungen 26, 27, die Sensorik 9 und die Steuer- und / oder Regelschaltung zur Steuerung und / oder Regelung der Antriebseinheit 2 sind auf einer gemeinsamen Trägervorrichtung in Form einer Platine 29 angeordnet. Teil des Steuermoduls 8' sind des Weiteren die Kontaktelemente 24, die das Steuermodul 8 mit der Antriebseinheit 2 verbinden, und die Kontaktelemente 25, die mit der Energiequelle für den Handgriff 1, insbesondere den Akkumulatoren 33, in Verbindung stehen. Die Kontaktelemente 24, 25 können zum Beispiel als Plankontakte, Federkontakte oder bevorzugt als Steckkontakte ausgeführt sein.

Steuermodul 8' umfasst des Weiteren einen Kühlkörper 36, der zum Beispiel aus einem oder mehreren Kühlblechen bestehen kann. Kühlkörper 36 ist aus einem Material mit hoher Wärmeleitfähigkeit, insbesondere aus Metall hergestellt. Er leitet die von den elektrischen und elektronischen Bauteilen des Steuermoduls 8' erzeugte Wärme ab und gibt zumindest einen Teil dieser Wärme an die Umgebung ab.

Gemäß der Ausführungsform nach Figur 4 sind die Platine 29, die Sensorik 9, die Steuer- und / oder Regelschaltung zur Steuerung und / oder Regelung der Antriebseinheit 2, zumindest jene Teile der Schaltungen 26, 27, die auf der Platine 29 befestigt sind, der Kühlkörper 36 und zumindest Teile der Kontakte 24, 25 in einem Gehäuse 28 aufgenommen. Bevorzugt besteht das Gehäuse 28 aus Kunststoff, besonders bevorzugt aus einem Vergussmaterial. Bevorzugt ist das Gehäuse 28 hermetisch verschlossen, so dass es alle in seinem Inneren angeordneten Bauteile derart von der Umgebung abdichtet, dass diese durch die während eines Reinigungs- oder Sterilisationsvorgangs herrschenden Umgebungsbedingungen in ihrer Funktion nicht beeinträchtigt werden. Die Herstellung des Gehäuses 28 aus Vergussmaterial hat insbesondere den Vorteil, dass im Steuermodul 8' im Wesentlichen keine Lufteinschlüsse oder größere Hohlräume mit Luft enthalten sind, so dass dadurch eine bessere Wärmeableitung aus dem Steuermodul 8' erfolgt. Das Gehäuse 28 kann auch als Trägervorrichtung dienen, so dass eine separate Trägervorrichtung, wie sie oben beschrieben wurde, nicht benötigt wird.

Das Zusammenfassen der oben angeführten Schaltungen, der Sensorik 9, der Kontaktelemente 24, 25 sowie gegebenenfalls weiterer Schaltungen, wie zum Beispiel einer Schaltung zur Temperaturüberwachung oder einer Schaltung zur Spannungsüberwachung in einem einzigen Steuermodul 8, 8' und deren Einkapselung in einem Gehäuse 28 hat insbesondere den Vorteil, dass die Verbindungsleitungen zwischen den einzelnen Schaltungen ebenfalls vollständig eingehaust sind. Es ist damit nicht mehr notwendig die Verbindungsleitungen zu isolieren, so dass keine Gefahr besteht, diese Isolierungen beim Vergießen zu zerstören, und es entfallen die Eintrittsstellen der Leitungen zu den einzelnen Schaltungen, die, wie weiter oben bereits beschrieben, besonders anfällig sind, undicht zu werden und über die Feuchtigkeit oder Reinigungsmittel zu den Schaltungen vordringen können. Anstelle einer großen Anzahl von Verbindungen und Kontakten zwischen den einzelnen, an unterschiedlichen Stellen im Handgriff angeordneten Schaltungen und Sensoren, die alle abgedichtet und isoliert werden müssen, gibt es bei der in einem einzigen Steuermodul 8, 8' zusammengefassten Steuervorrichtung somit nur noch zwei Schnittstellen, nämlich die Verbindung zur Antriebseinheit 2 über die Kontaktelemente 24 und die Verbindung zur Energiequelle über die Kontaktelemente 25.

Das Steuermodul 8, 8' hat somit zwei große Vorteile: Durch seine Kompaktheit erleichtert es insbesondere den Einbau in den Handgriff 1 und ermöglicht eine rasche und einfache Austauschbarkeit bei Reparaturen. Wird das Steuermodul 8, 8' zusätzlich mit einem Gehäuse 28 versehen, das hermetisch verschlossen ist und während eines Reinigungs- oder Sterilisationsvorgangs die Bauteile des Steuermoduls 8, 8' schützt, so ist damit ein Handgriff 1 geschaffen, dessen Steuervorrichtung oftmalige Sterilisation oder Reinigung unbeeinträchtigt übersteht, ohne dass vom Anwender vor oder nach dem Sterilisieren oder Reinigen zusätzliche Maßnahmen erforderlich sind.

Um die Zusammenfassung zu einem einstückigen Steuermodul 8, 8' und dessen Einbau in dem medizinischen Handgriff 1 zu ermöglich, ist es unter anderem notwendig, den Aufbau des Handgriffs 1 zu modifizieren. Insbesondere ist zumindest eines der beiden Magnetelement 6A, 6B so im Handgriff 1 anzuordnen, dass zumindest ein Teil des Steuermoduls 8, 8' in unmittelbarer Nähe des zumindest einen Magnetelements 6A, 6B positionierbar ist. Dies wird in dem Ausführungsbeispiel nach Figur 1 dadurch erreicht, dass der Sensormagnet 7 in einem Bereich des Funktionsabschnitts 11 angeordnet ist, an den der Griffabschnitt 10 anschließt. Wird nun ein Teil des Steuermoduls 8, 8', zum Beispiel das erste Ende 30, anschließend an den Sensormagnet 7 im Funktionsabschnitt 11 angebracht, so kann jener Teil des Steuermoduls 8, 8', der keinen Platz im Funktionsabschnitt 11 findet, im Hohlraum 32 des Griffabschnitts 10 positioniert werden.

Figur 2 zeigt eine Schnittdarstellung durch den Handgriff 1 gemäß Figur 1 entlang der Linie B-B, aus der die Anordnung des Steuermoduls 8 in unmittelbarer Nähe zum Sensormagneten 7 und jeweils zum Teil im Funktionsabschnitt 11 und im Griffabschnitt 10 gut zu erkennen ist. Die Sensorik 9 ist in den Ausführungsbeispielen gemäß den Figuren 1, 2 und 4 am äußersten Ende 30 des Steuermoduls 8, 8' angeordnet, so dass sie in unmittelbarer Nähe zum Sensormagneten 7 positionierbar ist. In diesen Figuren ist das erste Ende 30 des Steuermoduls 8, 8' zusätzlich durch Ausformung eines kreisbogenförmigen Rücksprungs 20 so beschaffen, dass es die Anordnung des Sensormagnets 7 und der Sensorik 9 in unmittelbarer Nähe zueinander begünstigt. Die Sensorik 9 umfasst bevorzugt drei Hall-Sensoren 37, die ungefähr in 45° oder 60°- Abständen rund um den kreisbogenförmigen Rücksprung 20 und den Sensormagnet 7 angeordnet sind.

In Figur 3 ist ein weiteres Ausführungsbeispiel des Steuermoduls 8" dargestellt. Steuermodul 8" umfasst eine Bohrung 21, in die der Sensormagnet 7 aufgenommen ist. Der Vorteil dieses Ausführungsbeispiels liegt darin, dass der Hersteller die Möglichkeit hat, mehr Hall-Sensoren 37 rund um den Sensormagnet 7 zu positionieren oder die Abstände zwischen den Hall-Sensoren 37 zu variieren, zum Beispiel die Sensoren 37 mit Abständen von 120° voneinander anzuordnen. Damit ist es möglich, eine exaktere Bestimmung der Ausrichtung des Magnetelements 6A, 6B zu erzielen.

Zur exakten Ausrichtung der Hall-Sensoren 37 in Bezug auf den Sensormagneten 7 werden bevorzugt die Kontakte 24 verwendet. Die Kontakte 24 und die Hall-Sensoren 37 sind in einem festgelegten Abstand voneinander auf der Platine 29 befestigt. Bei der Bestückung der Platine 29 mit den elektronischen und elektrischen Bauteilen wird mit sehr hoher Präzision vorgegangen, so dass die räumlichen Abstände zwischen verschiedenen Bauteilen von sehr hoher Konstanz sind. Diese präzise räumliche Anordnung der Bauteile und die konstante Entfernung zwischen den Bauteilen wird für die Ausrichtung der Hall-Sensoren 37 in Bezug auf den Sensormagneten 7 genutzt, in dem man das Steuermodul 8 über die Kontakte 24 im Handgriff 1 befestigt und dadurch gleichzeitig und ohne zusätzlichen Montageaufwand erreicht, dass die Hall-Sensoren 37 ausreichend exakt auf das Magnetelement 6A, 6B ausgerichtet sind.

Die Erfindung ist nicht auf das das beschriebene Ausführungsbeispiel beschränkt, sondern umfaßt alle Ausführungsmöglichkeiten, die das prinzipielle, sinngemäße Funktionsprinzip der Erfindung nicht verändern. Insbesondere kann die Antriebseinheit 2 auch als sensormagnetloser Elektromotor ausgeführt sein und die Position des Rotors direkt über den magnetischen oder mit Magneten versehenen Rotor bestimmt werden. In diesem Ausführungsbeispiel ist das Steuermodul 8 in unmittelbarer Nähe zum Rotor bzw. zum Rotormagneten anzuordnen.

## Patentansprüche

1. Medizinischer Handgriff (1) mit einer Antriebseinheit (2) zum Antrieb eines medizinischen Werkzeuges und mit einer Steuer- und / oder Ragelschaltung zur Steuerung und / oder Regelung der Antriebseinheit (2), wobei die Antriebseinheit (2) einen bürstenlosen Elektromotor (3) mit einem Stator (4) und mit einer Rotoreinheit (5) mit zumindest einem Magnetelement (6A, 6B) sowie eine Sensorik (9) zur Bestimmung der Ausrichtung des zumindest einen Magnetelements (6A, 6B) umfasst, wobei
die Sensorik (9) zur Bestimmung der Ausrichtung des zumindest einen Magnetelements (6A, 6B) und die Steuer- und 1 oder Regelschaltung In einem gemeinsamen Stauermodul (8, 8', 8") zusammengefasst sind, wobei das zumindest eine Magnetelement (6A, 6B) derart im medizinischen Handgriff (1) angeordnet ist,
dass zumindest ein Teil des Steuermoduls (8, 8', 8") in unmittelbarer Nähe des zumindest einen Magnetelements (6A, 6B) positionierbar ist und wobei die Sensorik (9) zur Bestimmung der Ausrichtung des zumindest einen Magnetelements (6A, 6B) derart in dem Steuermodul (8, 8'. 8") angeordnet ist, dass sie in unmittelbarer Nähe des zumindest einen Magnetelements (6A, 6B) positionierbar ist, wobei das Steuermodul (8. 8', 8") länglich ausgebildet ist und ein erstes, der Antriebseinheit (2) zugewandtes Ende (30) und ein zweites, einer Energiequelle zur Energlevorsorgung des Handgriffs (1) zugewandtes Ende (31) aufweist, **dadurch gekennzeichnet, dass** das erste Ende (30) mit Kontaktelementen (24), die das Steuermodul (8, 8', 8") mit Antriebseinheit (2) verbinden, zur Leitung von Antriebssignaten an die Antriebseinheit (2) und das zweite Ende (31) mit elektrischen Kontaktelementen (25) zur Verbindung des Steuermoduls (8, 8', 8") mit der Energiequelle versehen ist.

2. Medizinischer Handgriff (1) nach Anspruch 1, **dadurch gekennzeichnet, dass** das Steuermodul (8, 8', 8") so geformt ist, dass das zumindest eine Magnetelement (6A, 6B) und die Sensorik (9) zur Bestimmung der Ausrichtung des zumindest einen Magnetelements (6A, 6B) in unmittelbarer Nähe zueinander anordenbar sind, wobei das Steuermodul (8, 8', 8") insbesondere einen kreisbogenförmigen Rücksprung (20) oder einen stufenförmigen Rücksprung oder eine Bohrung (21) zur zumindest teilweisen Aufnahme des zumindest einen Magnetelements (6A, 6B) aufweist.

3. Medizinischer Handgriff (1) nach Anspruch 1 oder 2, **dadurch gekennzeichnet, dass** das Steuermodul (8. 8', 8") eine längliche, gewinkelte oder gebogene Form mit zumindest zwei Enden (30, 31) aufweist und dass die Sensorik (9) zur Bestimmung der Ausrichtung des zumindest einen Magnetelements (6A, 6B) an einem dieser Enden des Steuermoduls (8, 8', 8") angeordnet ist.

4. Medizinischer Handgriff (1) nach einem der vorhergehenden Ansprüche, **dadurch gekennzeichnet, dass**
der medizinische Handgriff (1) einen Griffabschnitt (10) und einen gewinkelt dazu angeordneten Funktionsabschnitt (11) umfasst, wobei das zumindest eine Magnetelement (6A, 6B) in einem der beiden Abschnitte (10, 11) angeordnet ist und das Steuermodul (8, 8', 8") zumindest teilweise auch in jenem Abschnitt (10, 11) aufgenommen ist, in dem das zumindest eine Magnetelement (6A, 6B) nicht angeordnet ist.

5. Medizinischer Handgriff (1) nach Anspruch 4, **dadurch gekennzeichnet, dass** das zumindest eine Magnetelement (6A, 6B) in einem Bereich des Funktionsabschnitts (11) angeordnet ist, an den der Griffabschnitt (10) anschließt, und dass das Steuermodul (8, 8', 8") zumindest teilweise im Griffabschnitt (10) aufgenommen ist.

6. Medizinischer Handgriff (1) nach einem der Ansprüche 1 bis 5, **dadurch gekennzeichnet, dass**
der medizinische Handgriff (1) eine Werkzeugaufnahme für das medizinische Werkzeug oder eine Anschlussvorrichtung (12) für eine Werkzeugaufnahme aufweist, dass die Rotoreinheit (5) einen Rotor (13) und einen Sensormagneten (7) umfasst und dass der Sensormagnet (7) und das Steuermodul (8, 8', 8") zwischen dem Rotor (13) und der Werkzeugaufnahme bzw. der Anschlussvorrichtung (12) angeordnet sind.

7. Medizinischer Handgriff (1) nach einem der vorhergehenden Ansprüche, **dadurch gekennzeichnet, dass**
das Steuermodul (8, 8', 8") eine Trägervorrichtung umfasst, insbesondere eine Trägerplatte oder eine Platine (29), auf der zumindest die Steuer- und / oder Regelschaltung und die Sensorik (9) befestigt sind.

8. Medizinischer Handgriff (1) nach einem der vorhergehenden Ansprüche, **dadurch gekennzeichnet, dass**
das Steuermodul (8, 8', 8") zumindest teilweise von einem Gehäuse (28), insbesondere von einem hermetisch verschlossenen Gehäuse (28), umgeben ist.

9. Medizinischer Handgriff (1) nach Anspruch 8, **dadurch gekennzeichnet, dass** das Gehäuse (28) so beschaffen ist, dass die Funktionsfähigkeit der im Inneren des Gehäuses (28) befindlichen Bauelemente auch nach oftmaliger Reinigung oder Sterilisation aufrecht erhaltbar ist.

10. Medizinischer Handgriff (1) nach Anspruch 8 oder 9, **dadurch gekennzeichnet, dass** das Gehäuse (28) aus Vergussmaterial, zum Beispiel aus Kunstharz, insbesondere aus Epoxyd- oder Silikonharz, hergestellt ist.

11. Medizinischer Handgriff (1) nach einem der vorhergehenden Ansprüche, **dadurch gekennzeichnet, dass**
in dem Steuermodul (8, 8', 8") des Weiteren zumindest Teile der Schaltung zur Auswahl der Antriebsdrehzahl (27) und / oder zumindest Teile der Schaltung zur Auswahl der Drehrichtung (26) des Elektromotors (3) und / oder zumindest Teile einer Schaltung zur Temperaturüberwachung Wärme abgebender Bauteile des Handgriffs (1) und / oder zumindest Teile einer Schaltung zur Spannungsüberwachung spannungsführender Bauteil des Handgriffs (1) vorgesehen sind.

12. Medizinischer Handgriff (1) nach einem der vorhergehenden Ansprüche, **dadurch gekennzeichnet, dass**
das Steuermodul (8, 8', 8") einen Kühlkörper (36) umfasst.

13. Medizinischer Handgriff (1) nach einem der vorhergehenden Ansprüche, **dadurch gekennzeichnet, dass**
die Kontaktelemente (24, 25) als Plankontakte, Federkontakte oder als Steckkontakte ausgebildet sind.

14. Medizinischer Handgriff (1) nach einem der vorhergehenden Ansprüche, **dadurch gekennzeichnet, dass**
die Energiequelle eine Batterie oder einen Akkumulator (33) umfasst, wobei die Kontaktelemente (25) am zweiten Ende (31) des Steuermoduls (8, 8', 8") direkt mit der Batterie oder dem Akkumulator (33) verbunden sind.

15. Medizinischer Handgriff (1) nach einem der vorhergehenden Ansprüche, **dadurch gekennzeichnet, dass**
das Steuermodul (8, 8', 8") durch die Kontaktelemente (24) zur Leitung von Antriebssignalen an die Antriebseinheit (2) im Handgriff (1) befestigt ist, wodurch gleichzeitig die Hall-Sensoren (37) der Sensorik (9) exakt auf das Magnetelement (6A, 6B) ausgerichtet sind.

## Claims

1. A medical handle (1) with a drive unit (2) for driving a medical tool and a control and/or regulating circuit for controlling and/or regulating the drive unit (2), wherein the drive unit (2) comprises a brushless electric motor (3) with a stator (4) and a rotor unit (5) with at least one magnetic element (6A, 6B) as well as a sensor system (9) for determining the alignment of the at least one magnetic element (6A, 6B), wherein the sensor system (9) for detecting the alignment of the at least one magnetic element (6A, 6B) and the control and/or regulating circuit are combined in a joint control module (8, 8', 8"), wherein the at least one magnetic element (6A, 6B) is arranged in the medical handle (1) in such a way that at least a part of the control module (8, 8', 8") can be positioned in the immediate vicinity of the at least one magnetic element (6A, 6B) and wherein the sensor system (9) for determining the alignment of the at least one magnetic element (6A, 6B) is arranged in the control module (8, 8', 8") in such a way that it can be positioned in the immediate vicinity of the at least one magnetic element (6A, 6B), wherein the control module (8, 8', 8") has an elongated shape and comprises a first end (30) turned toward the drive unit (2) and a second end (31) turned toward a energy source for supplying energy to the handle (1), **characterized in that**
the first end (30) is provided with contact elements (24) which connect the the control module (8, 8', 8") to the drive unit (2) for the supply of drive signals to the drive unit (2) and the second end (31) is provided with electrical contact elements (25) for connecting the control module (8, 8', 8") to the energy source.

2. The medical handle (1) according to claim 1, **characterized in that** the control module (8, 8', 8") is shaped in such a way that the at least one magnetic element (6A, 6B) and the sensor system (9) for detecting the alignment of the at least one magnetic element (6A, 6B) can be arranged in direct proximity to one another, wherein the control module (8, 8', 8") comprises in particular an arc-shaped setback (20) or a step-shaped setback or a bore (21) for at least partially accommodating the at least one magnetic element (6A, 6B).

3. The medical handle (1) according to claim 1 or 2, **characterized in that** the control module (8, 8', 8") has an elongated, angled or curved shape with at least two ends (30, 31) and the sensor system (9) for detecting the alignment of the at least one magnetic element (6A, 6B) is arranged on one of these ends of the control module (8, 8', 8").

4. The medical handle (1) according to any one of the preceding claims, **characterized in that**
the medical handle (1) comprises a grip section (10) and a function section (11) arranged at an angle to the former, wherein the at least one magnetic element (6A, 6B) is arranged in one of the two sections (10, 11) and the control module (8, 8', 8") is accommodated at least partially in the section (10, 11) in which the at least one magnetic element (6A, 6B) is not arranged.

5. The medical handle (1) according to claim 4, **characterized in that**
the at least one magnetic element (6A, 6B) is arranged in an area of the function section (11) to which the grip section (10) is adjacent, and the control module (8, 8', 8") is accommodated at least partially in the grip section (10).

6. The medical handle (1) according to any one of claims 1 - 5, **characterized in that**
the medical handle (1) comprises a tool receptacle for the medical tool or a connecting device (12) for a tool receptacle, the rotor unit (5) comprises a rotor (13) and a sensor magnet (7) and the sensor magnet (7) and the control module (8, 8', 8") are arranged between the rotor (13) and the tool receptacle and/or the connecting device (12).

7. The medical handle (1) according to any one of the preceding claims, **characterized in that**
the control module (8, 8', 8") comprises a carrier device, in particular a carrier plate or a circuit board (29), on which at least the control and/or regulating circuit and the sensor system (9) are mounted.

8. The medical handle (1) according to any one of the preceding claims, **characterized in that**
the control module (8, 8', 8") is surrounded at least partially by a housing (28), in particular by a hermetically sealed housing (28).

9. The medical handle (1) according to claim 8, **characterized in that**
the housing (28) is designed so that the operability of the components in the interior of the housing (28) can be maintained even after frequent cleaning or sterilization.

10. The medical handle (1) according to claim 8 or 9, **characterized in that** the housing (28) is made of a casting material, for example of a resin, in particular of a epoxy or silicone resin.

11. The medical handle (1) according to any one of the preceding claims, **characterized in that**
additionally at least parts of the circuit for selecting the drive rotational speed (27) and/or at least parts of the circuit for selecting the direction of rotation (26) of the electric motor (3) and/or at least parts of a circuit for monitoring the temperature of heat-emitting components of the handle (1), and/or at least parts of a circuit for monitoring the voltage of voltage-carrying parts of the handle (1) are provided in the control module (8, 8', 8").

12. The medical handle (1) according to any one of the preceding claims, **characterized in that**
the control module (8, 8', 8") comprises a heat sink (36).

13. The medical handle (1) according to any one of the preceding claims, **characterized in that**
the contact elements (24, 25) are designed as flat contacts, spring contacts or as plug contacts.

14. The medical handle (1) according to any one of the preceding claims, **characterized in that**
the energy source comprises a battery or an accumulator (33), wherein the contact elements (25) at the second end (31) of the control module (8, 8', 8") are directly connected to the battery or the accumulator (33).

15. The medical handle (1) according to any one of the preceding claims, **characterized in that**
the control module (8, 8', 8") is mounted in the handle (1) via the contacts (24) for the supply of drive signals to the drive unit (2), so that at the same time the Hall sensors (37) of the sensor system (9) are aligned exactly with the magnetic element (6A, 6B).

## Revendications

1. Poignée médicale (1) comprenant une unité d'entraînement (2) pour entraîner un outil médical et un montage de commande et/ou réglage pour diriger et/ou régler l'unité d'entraînement (2), dans laquelle l'unité d'entraînement (2) comprend un moteur électrique sans brosse (3) avec un stator (4) et une unité de rotor (5) comprenant au moins un élément magnétique (6A, 6B) ainsi qu'un système de capteurs (9) pour déterminer l'alignement de l'au moins un élément magnétique (6A, 6B), sachant que le système de capteurs (9) pour déterminer l'alignement de l'au moins un élément magnétique (6A, 6B) et le montage de commande et/ou réglage sont réunis dans un module de commande commun (8, 8', 8"), sachant que l'au moins un élément magnétique (6A, 6B) est ainsi placé dans la poignée médicale (1) qu'au moins une partie du module de commande (8, 8', 8") peut être positionnée dans le voisinage direct de l'au moins un élément magnétique (6A, 6B) et sachant que le système de capteurs (9) pour déterminer l'alignement de l'au moins un élément magnétique (6A, 6B) est ainsi placé dans le module de commande (8, 8', 8") qu'il peut être positionné dans le voisinage direct de l'au moins un élément magnétique (6A, 6B), sachant que le module de commande (8, 8', 8") est oblong et présente une première extrémité (30) tournée vers l'unité d'entraînement (2) et une deuxième extrémité (31) tournée vers une source d'énergie pour alimenter la poignée (1) en énergie, **caractérisée en ce que**
la première extrémité (30) est munie d'éléments de contact (24) reliant le module de commande (8, 8', 8") à l'unité d'entraînement (2) pour amener des signaux d'entraînement à l'unité d'entraînement (2) et la deuxième extrémité (31) est munie d'éléments de contact électriques (25) pour relier le module de commande (8, 8', 8") à la source d'énergie.

2. Poignée médicale (1) selon la revendication 1, **caractérisée en ce que** le module de commande (8, 8', 8") est ainsi formé que l'au moins un élément magnétique (6A, 6B) et le système de capteurs (9) pour déterminer l'alignement de l'au moins un élément magnétique (6A, 6B) peuvent être placés dans leur voisinage immédiat l'un de l'autre, sachant que le module de commande (8, 8', 8") présente en particulier un retrait (20) en forme d'arc de cercle ou un retrait en forme de cran ou un perçage (21) pour recevoir au moins en partie au moins un élément magnétique (6A, 6B).

3. Poignée médicale (1) selon la revendication 1 ou 2, **caractérisée en ce que** le module de commande (8, 8', 8") a une forme oblongue, coudée ou cintrée avec aux moins deux extrémités (30, 31) et que le système de capteurs (9) pour déterminer l'alignement de l'au moins un élément magnétique (6A, 6B) est placé sur l'une de ces extrémités du module de commande (8, 8', 8").

4. Poignée médicale (1) selon l'une des revendications précédentes, **caractérisée en ce que**
la poignée médicale (1) comprend un tronçon de poignée (10) et un tronçon de fonctionnement (11) coudé par rapport à celui-ci, sachant que l'au moins un élément magnétique (6A, 6B) est placé dans au moins un des deux tronçons (10, 11) et que le module de commande (8, 8', 8") est reçu au moins partiellement aussi dans le tronçon (10, 11) dans lequel l'au moins un élément magnétique (6A, 6B) n'est pas placé.

5. Poignée médicale (1) selon la revendication 4, **caractérisée en ce que** l'au moins un élément magnétique (6A, 6B) est placé dans une zone du tronçon de fonction (11) sur laquelle le tronçon de poignée (10) se rattache et que le module de commande (8, 8', 8") est reçu au moins partiellement dans le tronçon de poignée (10).

6. Poignée médicale (1) selon l'une des revendications 1 à 5, **caractérisée en ce que** la poignée médicale (1) présente un emmanchement d'outil pour l'outil médical ou un dispositif de connexion (12) pour un emmanchement d'outil, que l'unité de rotor (5) comprend un rotor (13) et un aimant de capteur (7) et **en ce que** l'aimant de capteur (7) et le module de commande (8, 8', 8") sont placés entre le rotor (13) et l' emmanchement d'outil respectivement le dispositif de connexion (12).

7. Poignée médicale (1) selon l'une des revendications précédentes, **caractérisée en ce que**
le module de commande (8, 8', 8") comprend un dispositif de support, en particulier une plaque porteuse ou une platine (29), sur laquelle au moins le montage de commande et/ou de réglage et le système de capteurs (9) sont fixés.

8. Poignée médicale (1) selon l'une des revendications précédentes, **caractérisée en ce que**
le module de commande (8, 8', 8") est entouré au moins en partie d'un boîtier (28), en particulier d'un boîtier (28) fermé hermétiquement.

9. Poignée médicale (1) selon la revendication 8, **caractérisée en ce que** le boîtier (28) est ainsi prévu que la capacité de fonctionnement des composants se trouvant à l'intérieur du boîtier (28) puisse être maintenue également même en cas de nettoyage ou stérilisation fréquents.

10. Poignée médicale (1) selon la revendication 8 ou 9, **caractérisée en ce que** le boîtier (28) est fabriqué en matériau fondu, par exemple en résine synthétique, en particulier en résine epoxy ou de silicone.

11. Poignée médicale (1) selon l'une des revendications précédentes, **caractérisée en ce que**
dans le module de commande (8, 8', 8") sont prévues en outre au moins des parties du montage pour sélectionner le nombre de tours (27) et/ou au moins des parties du montage pour sélectionner le sens de rotation (26) du moteur électrique (3) et/ou au moins des parties d'un montage pour surveiller la température des composants de la poignée (1) libérant de la chaleur et/ou au moins des parties d'un montage pour surveiller la tension des composants de la poignée (1) sous tension.

12. Poignée médicale (1) selon l'une des revendications précédentes, **caractérisée en ce que**
le module de commande (8, 8', 8") comprend un refroidisseur (36).

13. Poignée médicale (1) selon l'une des revendications précédentes, **caractérisée en ce que**
les éléments de contact (24, 25) sont des contacts plans, des contacts à ressort ou des contacts à broche.

14. Poignée médicale (1) selon l'une des revendications précédentes, **caractérisée en ce que**
la source d'énergie comprend une batterie ou un accumulateur (33) sachant que les éléments de contact (25) sont reliés sur la deuxième extrémité (31) du module de commande (8, 8', 8") directement à la batterie ou à l'accumulateur (33).

15. Poignée médicale (1) selon l'une des revendications précédentes, **caractérisée en ce que**
le module de commande (8, 8', 8") est fixé dans la poignée (1) par les éléments de contact (24) pour amener des signaux d'entraînement à l'unité d'entraînement (2), ce par quoi parallèlement les capteurs à effet Hall (37) du système de capteurs (9) sont alignés exactement sur l'élément magnétique (6A, 6B).
